(19) Europäisches Patentamt
European Patent Office
Office européen des brevets

(11) Veröffentlichungsnummer: **0 155 625**
**A2**

(12) # EUROPÄISCHE PATENTANMELDUNG

(21) Anmeldenummer: **85102795.3**

(22) Anmeldetag: **12.03.85**

(51) Int. Cl.$^4$: **A 61 K 9/50**
C 07 H 13/06, A 61 K 31/70

(30) Priorität: **21.03.84 DE 3410238**

(43) Veröffentlichungstag der Anmeldung:
**25.09.85 Patentblatt 85/39**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schlossmann, Klaus, Dr.**
**In der Beek 116**
**D-5600 Wuppertal 1(DE)**

(72) Erfinder: **Zembrod, Alfred, Dr.**
**Nittumer Weg 60**
**D-5060 Bergisch-Gladbach 2(DE)**

(72) Erfinder: **Lockhoff, Oswald, Dr.**
**Morgengraben 14**
**D-5000 Köln 80(DE)**

(72) Erfinder: **Kroll, Hein-Peter, Dr.**
**Pahlkestrasse 96**
**D-5600 Wuppertal 1(DE)**

(72) Erfinder: **Metzger, Karl Georg, Dr.**
**Pahlkestrasse 75**
**D-5600 Wuppertal 1(DE)**

(72) Erfinder: **Opitz, Hans-Georg, Dr.**
**2200 Powell Street P.O. Box 8817**
**Emeryville California 94662(US)**

(72) Erfinder: **Paessens, Arnold, Dr.**
**Stresemannstrasse 56**
**D-5657 Haan 1(DE)**

(72) Erfinder: **Schaller, Klaus, Dr.**
**Am Sonnenschein 38**
**D-5600 Wuppertal 1(DE)**

(72) Erfinder: **Streissle, Gert, Dr.**
**Gellertweg 22**
**D-5600 Wuppertal 1(DE)**

(72) Erfinder: **Stünkel, Klaus, Dr.**
**Am Eckbusch 55**
**D-5600 Wuppertal 1(DE)**

(72) Erfinder: **Zeiler, Hans-Joachim, Dr.**
**Elsbeeker Strasse 46**
**D-5620 Velbert 15(DE)**

(54) **Glycolipid-ähnliche Substanzen in Liposomenform.**

(57) Die vorliegende Erfindung betrifft glycolipidähnliche Substanzen in Liposomenform, Verfahren zu ihrer Herstellung sowie ihre Verwendung als Arzneimittel .

Die Verbindungen haben die allgemeine Formel

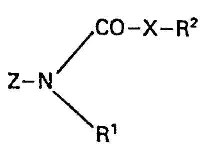

(I)

in welcher $R^1$, $R^2$, X und Z die in der Beschreibung angegebene Bedeutung besitzen.

EP 0 155 625 A2

BAYER AKTIENGESELLSCHAFT 5090 Leverkusen, Bayerwerk

Konzernverwaltung RP
Patentabteilung Ad/ABc

## Glycolipid-ähnliche Substanzen in Liposomenform

Die Erfindung betrifft glycolipidähnliche Substanzen in Liposomenform, Verfahren zu ihrer Herstellung sowie ihre Verwendung als Arzneimittel.

Unter Liposomen werden erfindungsgemäß künstliche Teilchen mit einer Doppelschicht-Membranstruktur verstanden, analog der bekannten Anordnung von Phospholipiden in biologischen Membranen. Die Liposomen werden durch die membranartigen Strukturen selbst gebildet. Die Membranfläche kann dabei in sich geschlossen sein (Kugelform, langgestreckte oder abgeplattete Ellipsoide, helikale Formen), sie kann aber auch offen sein (planar, schlauchförmig oder U-förmig). Die Membranflächen können auch mehrfach übereinander oder konzentrisch zueinander angeordnet sein (entsprechend dem konventionellen Begriffspaar für - normalerweise - kugelförmige Liposomen: unilamellar oder multilamellar). Unter Liposomen werden auch Aggregate dieser Membran-Grundformen verstanden.

Unter glycolipidähnlichen Substanzen werden erfindungsgemäß glycosylierte Carbonsäureamid-Derivate und Kohlensäurederivate verstanden. Es sind dies Verbindungen der allgemeinen Formel I

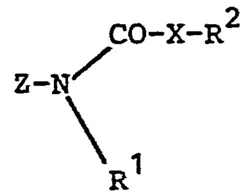

(I)

Hierin bedeutet

$R^1$ einen gegebenenfalls substituierten geradkettigen oder verzweigten, gesättigten oder einfach oder mehrfach ungesättigten Alkyl- bzw. Alkenylrest mit bis zu 30 Kohlenstoffatomen,

$R^2$ Wasserstoff oder einen gegebenenfalls substituierten geradkettigen oder verzweigten, gesättigten oder ein- oder mehrfach ungesättigten Kohlenwasserstoffrest mit bis zu 30 Kohlenstoffatomen,

X eine Methylengruppe, ein Sauerstoffatom, ein Schwefelatom oder eine Gruppe NR, in der R für Wasserstoff oder eine Alkylgruppe mit bis zu 20 Kohlenstoffatomen steht,

Z einen gegebenenfalls phosphorylierten Glykosylrest, der über das anomere Kohlenstoffatom an den Amidstickstoff gebunden ist.

$R^2$ in der Bedeutung Kohlenwasserstoffrest steht im allgemeinen für einen Alkyl-, Alkenyl-, Cycloalkyl-, Cycloalkenyl- oder Arylrest, wobei diese auch gemeinsam auftreten können, z.B. als Arylalkyl, Alkylaryl, Alkylcycloalkyl, Arylcycloalkyl und den entsprechenden Kombinationen mit ungesättigten Alkyl- und Cycloalkylteilen.

In den Resten $R^1$ und $R^2$ in der Bedeutung Alkyl oder Alkenyl können auch einzelne, im allgemeinen bis zu 5, vorzugsweise 1 oder 2 Methylen- oder Methingruppen durch O, S oder N ersetzt sein. Bei dieser Unterbrechung der Kette durch N trägt dieses Stickstoffatom entweder H oder einen Alkylrest mit bis zu 20 Kohlenstoffatomen oder einen CO-Alkylrest, wobei diese Alkylgruppe bis zu 20 C-Atome vorzugsweise jeweils bis zu 10 C-Atomen aufweist.

Bevorzugt stellt $R^1$ einen Alkylrest mit einem bis 21 Kohlenstoffatomen, vorzugsweise 10 bis 21 C-Atomen dar. Beispielhaft für diese Reste $R^1$ seien hier genannt Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Didecyl, Methyldecyl, Methyloctadecyl, Decylhexadecyl.

Ungesättigte Reste sind beispielsweise Ethenyl, Propenyl-1, Propenyl-2, i-Butenyl, Butenyl-1, Butenyl-2, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Decenyl, 5-Decenyl, 9-Decenyl, 8-Heptadecenyl, 1,3-Butadienyl, 1,3-Pentadienyl, 1,4-Pentadienyl, 2,4-Pentadienyl, 8,11-Heptadecadienyl, 8,11,14-Heptadecantrienyl. Im allgemeinen sind die längerkettigen, ungesättigten Reste bevorzugt, speziell die ein- oder zweifach ungesättigten Alkenyle mit 7-21 C-Atomen.

Die ungesättigten Kohlenwasserstoffreste können dabei als reine cis- oder trans-Isomere oder auch als Isomerengemische vorliegen.

Beispiele, in welchen $R^2$ in Formel I für einen geradkettigen oder verzweigten, gegebenenfalls einfach oder mehrfach ungesättigten Alkylrest steht, sind die für $R^1$ genannten.

Besonders seien genannt Methyl, Propyl, Hexyl, Decyl, Undecyl, Dodecyl, Tridecyl, Tetradecyl, Pentadecyl, Hexadecyl, Heptadecyl, Octadecyl, Nonadecyl, Docosyl, Myricyl, Ethylhexyl, Isobutyl, Propenyl, Octenyl, Hexadienyl, Docosenyl, Dimethylhexenyl und 2-(Cyclohexyl)-ethyl. Die ungesättigten Kohlenwasserstoffreste können als reine cis- oder trans-Isomere oder als Isomerengemisch vorliegen.

Beispiele für $R^2$ in der Bedeutung Cycloalk(en)yl sind Cyclopropyl, Cyclobutyl, Cyclopentyl, Cyclohexyl, Cycloheptyl, Adamantyl, Cyclobutylcyclopentyl, Cyclopentylcyclopentyl, Cyclohexylcyclopentyl, Cyclohexylcyclohexyl, Cyclohexyladamantyl, Adamantylcyclohexyl, Dicyclohexylcyclopentyl, Decahydronaphthyl.

Beispielhaft für Alkylcycloalkylreste $R^2$ seien genannt: Methylcyclopentyl, Ethylcyclopentyl, n-Propylcyclopentyl, i-Propylcyclopentyl, Butylcyclopentyl, Octylcyclopentyl, Methylcyclohexyl, Ethylcyclohexyl, Propylcyclohexyl, Butylcyclohexyl, Hexylcyclohexyl, Decylcyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexylhexyl, Cyclohexyldecyl, Cyclopentylcyclohexylethyl, Cyclohexylcyclopentylethyl und Cyclohexylcyclohexylethyl.

In den genannten Beispielen, in denen die Cycloalkylreste zweifach substituiert sind, können die Substituenten sowohl cis als auch trans zueinander angeordnet sein.

$R^2$ in der Bedeutung Aryl steht bevorzugt für aromatische Kohlenwasserstoffreste mit 6, 10 oder 12 Kohlenstoffatomen. Beispiele hierfür sind Phenyl, Biphenyl und Naphthyl.

Die Arylreste können substituiert sein, vorzugsweise durch $C_1$-$C_4$-Alkyl, $C_1$-$C_4$-Alkoxy, Halogen- vorzugsweise F, Cl und Br und Nitro. Im Falle der Substitution kommen 1-3, bei Halogen 1-5 Substituenten in Betracht. Beispiele hierfür sind: Nitrophenyl, Methoxyphenyl, Chlorphenyl, Dichlorphenyl, Hydroxynaphthyl, Benzyl, Methoxybenzyl, Phenylethyl, Phenylhexyl, Phenyldodecyl, p-Methoxyphenylhexyl, Naphthylbutyl.

Beispiele in denen die Reste $R^1$ und/oder $R^2$ in Formel (I) durch Sauerstoff-, Schwefel- und/oder Stickstoffatome unterbrochene Kohlenwasserstoffreste darstellen, sind

Methoxyethyl, Methoxyethoxyethyl, Dimethylaminoethyloxyethyl, Dibutylaminohexyl, N-Methylaminodecyl, Methoxybutyl oder Butoxypropyl. Ein durch N und O unterbrochener Rest ist beispielsweise 2-(N-morpholino)-ethyl und als Beispiel für einen halogensubstituierten Kohlenwasserstoffrest sei Trifluormethylethyl genannt.

Die Reste $R^1$ und $R^2$ können substituiert sein, vorzugsweise 1-3fach. Bevorzugte Substituenten hierfür sind $C_6$-, $C_{10}$- oder $C_{12}$-Aryl, Halogen, vorzugsweise Cl und Br, Amino, $C_1$-$C_6$-Alkylamino, Di-$C_1$-$C_6$-alkylamino, Oxo, OH, $C_1$-$C_6$-Alkoxy, SH, $C_1$-$C_6$-Alkylmercapto, $C_1$-$C_6$-Alkyl-COO und $C_1$-$C_6$-Alkyl-OC-NH.

Beispiele für derart substituierte Reste $R^1$ und $R^2$ sind Mercaptoethyl, ß-Hydroxytridecyl, $\omega$-Hydroxyheptadecenyl, Oxobutyl, Aminodecyl, Chlorethyl, Fluormethyl.

Z in Formel I bedeutet einen gegebenenfalls phosphorylierten Glykosylrest, der immer über das anomere Kohlenstoffatom an den Amidstickstoff gebunden ist, wobei unter Glykosylreste erfindungsgemäß besonders verstanden werden Mono-, Di- und Oligosaccharidreste, besonders Mono- und Disaccharide, in denen gegebenenfalls eine oder mehrere Hydroxylgruppen durch Aminogruppen, Acylamidogruppen, Azidogruppen, Wasserstoff, Nitro, Thiolgruppen oder Niedrigalkoxy oder Halogen ersetzt sein können und die Glykosylreste auch in Form der entsprechenden Ulosen, Ulosederivate, Uronsäuren oder von Uronsäurederivaten vorliegen können.

Die Glykosylreste Z in Formel I liegen erfindungsgemäß bevorzugt in der Pyranosyl- oder der Furanosylform vor,

wobei die betreffenden Monosaccharid-, Disaccharid oder Oligosaccharidreste bevorzugt aus Pentosen, Hexosen und Heptosen aufgebaut sind.

Beispiele für Monosaccharidreste sind Glucopyranosyl-, Galactopyranosyl-, Mannopyranosyl-, Glucofuranosyl-, Ribofuranosyl, Arabinopyranosyl- oder auch Lyxopyranosyl- oder auch D-Glycero-D-gluco-hepto-pyranosylreste. Als Beispiel für Di- und Oligosaccaridreste können genannt werden Maltosyl-, Maltotriosyl- , Maltotetraosyl-, Lactosyl-, Cellobiosyl-, Melibiosyl- oder 6-O-(α- oder ß-Ribofuranosyl)-Glucopyranosylreste, d.h. also Kohlenhydratsysteme, die aus Zuckern unterschiedlicher C-Zahl aufgebaut sein können und bei denen die Zucker in der Pyranose und/oder Furanoseform vorliegen können. Die glykosidischen Bindungen zwischen den einzelnen Zuckerbausteinen können in der α- und/oder ß-Form vorliegen und die glykosidische Verknüpfung der einzelnen Zuckerbausteine kann von einem anomeren Kohlenstoffatom ausgehend sowohl über die primäre OH-Gruppe als auch über eine der sekundären Hydroxylgruppen des als Aglykon fungierenden Saccharidteils erfolgen.

Als Beispiele für Mono-, Di- und Oligosaccharidreste, in denen eine oder mehrere OH-Gruppen durch Aminogruppen, Acylamidogruppen, Azidogruppen, Wasserstoff, Nitro, Thiolgruppen, Niedrigalkoxy oder Halogen ersetzt sind, seien genannt 2-Acetylamido-2-desoxyglucopyranosyl-, 2-Amino-2-desoxy-glucopyranosyl-, 4-Azido-4-desoxy-glucopyranosyl-, 4-Stearoylamido-4-desoxy-D-glucopyranosyl-, 4-Dodecoylamido-4-desoxy-D-glucopyranosyl-, 6-Hexa-

decanoylamido-6-desoxy-D-galactopyranosyl-, 2,6-Diamino-2,6-didesoxyglucopyranosyl-, 6,6'-Diamino-6,6'-didesoxymaltosyl-, 6-Amino-6,6'-didesoxylactosyl, 6-Desoxymannopyranosyl-, 2-Desoxyribofuranosyl-, Fucosyl, 5-Fluor-5-desoxyribofuranosyl-, 6-O-Methylglucopyranosyl, 6-Desoxy-6-thio-glucopyranosyl, 3-Desoxy-3-nitrogalactopyranosyl.

Liegen die Glykosylreste in Form von Uronsäuren oder Uronsäurederivaten vor, so handelt es sich um Glykuronsäuren mit freier Carboxylgruppe oder mit durch Alkyl veresterter Carboxylgruppe oder um Glykuronamidderivate mit unsubstituiertem oder substituiertem Stickstoffatom. Beispiele für entsprechende Zucker sind Galacturonsäure, Glucuronsäuremethylester oder auch N-Dodecylglucuronamid.

Die Glycosylreste können auch an einer oder zwei Hydroxygruppen mit Phosphorsäure oder Phosphorsäureestern esterartig verbunden sein. Unter Phosphorsäureestern sind die Gruppen

$$-\overset{\overset{\displaystyle O}{\|}}{\underset{\underset{\displaystyle O\text{-Alkyl}}{|}}{P}}\text{-OH} \quad \text{oder} \quad -\overset{\overset{\displaystyle O}{\|}}{\underset{\underset{\displaystyle O\text{-Alkyl}}{|}}{P}}\text{-O-Alkyl}$$

zu verstehen, wobei unter Alkyl ein Alkylrest mit bis zu 20 Kohlenstoffatomen zu verstehen ist.

Die Verbindungen der Formel I werden hergestellt, indem man die von Z in Formel I umfaßten Zucker entweder in

freier, d.h. ungeschützter Form oder in Form geschützter, gegebenenfalls aktivierter Derivate zunächst umsetzt mit einer Aminoverbindung $R^1$-$NH_2$ zu einem Glycosylamin der Formel II

$$Z\text{-}NH\text{-}R^1 \qquad (II)$$

Die Glycosylamine der Formel II werden dann in einem zweiten Reaktionsschritt entweder mit Carbonsäurederivaten der Formel III

$$R^2\text{-}CH_2\text{-}CO\text{-}Y \qquad (III)$$

umgesetzt, wobei $R^2$ die oben angeführte Bedeutung hat und Y für eine bei Acylierungsreaktionen übliche, aktivierende Gruppe wie Halogen oder -O-CO-$R^2$ oder -O-CO-O-$R^2$ steht. Auf diese Weise gelangt man zu Glycosylamiden gemäß Formel I mit X = $CH_2$.

Die Acylierung der Glycosylamine gemäß Formel I mit Halogenameisensäureestern der Formel IV

$$R^2\text{-}O\text{-}CO\text{-}Y \qquad (IV)$$

wobei $R^2$ die oben angegebene Bedeutung hat und Y für Halogen, bevorzugt Chlor steht, führt zu Glycosylurethanen gemäß Formel I mit X = O.

Die Reaktion der im ersten Reaktionsschritt erhaltenen Glycosylamine der Formel II mit Thiokohlensäurechlorid-S-estern der Formel V

$$R^2\text{-}S\text{-}CO\text{-}Cl \qquad (V)$$

wobei $R^2$ die oben angegebene Bedeutung hat, führt zu S-substituierten Thiocarbamaten der Formel I, wobei X in Formel I für ein Schwefelatom steht.

Werden die im ersten Reaktionsschritt erhaltenen Glycosylamine der Formel II im zweiten Reaktionsschritt mit einem organischen Isocyanat der Formel VI

$$R^2\text{-NCO} \qquad \text{(VI)}$$

umgesetzt, wobei $R^2$ die oben angeführte Bedeutung hat, erhält man Harnstoffe gemäß Formel I mit X = NH.

Die Darstellung der Glycosylamine ist in der Literatur beschrieben (vgl. G.P.ELLIS und J. HONEYMAN, Advances in Carbohydrate Chemistry 10 (1955) 95).

Zur Darstellung der Verbindungen gemäß Formel I werden die von Formel II umfaßten Glycosylamine in einem inerten organischen Lösungsmittel mit den in den Formeln III, IV, V und VI genannten Acylierungsmitteln aus der Carbonsäure- bzw. Kohlensäurereihe umgesetzt, gegebenenfalls unter Zusatz von organischen oder anorganischen Basen.

Als Lösungsmittel kommen solche in Frage, in denen sich zumindest entweder die Ausgangsprodukte oder die Endprodukte der Reaktion lösen. Dies sind z.B. Alkohole wie Methanol, Ethanol, Propanol, Isopropanol, Butanol, Octanol, Ethoxyethanol, Glycol oder Glycerin oder Ketone wie Aceton, Methylethylketon, Diisopropylketon, oder Ether wie Diethylether, Diisopropylether, Tetrahydro-

0155625

furan oder Dioxan oder Ester wie Essigester oder Acetessigester oder Nitrile, wie Acetonitril oder aromatische oder aliphatische Kohlenwasserstoffe, wie Toluol, Benzol, Hexan, Cyclohexan, Petrolether oder auch Dimethylformamid. Die genannten Lösungsmittel können allein oder im Gemisch untereinander oder im Gemisch mit Wasser verwendet werden.

Die Reaktionstemperaturen der Acylierungsreaktionen, die zu Verbindungen der Formel I führen, liegen im Bereich zwischen -78°C und +120°C, bevorzugt zwischen 0°C und +50°C.

Die in den Formeln III, IV, V und VI beschriebenen Carbonsäure- bzw. Kohlensäurederivate werden in der Menge von 1 bis 10 Äquivalenten mit den Glycosylaminen der Formel II eingesetzt, bevorzugt in der Menge von 1 bis 3 Äquivalenten.

Als Basen bei den Acylierungsreaktionen sind anorganische wie Natriumcarbonat oder organische wie Triethylamin oder Pyridin geeignet.

Falls eine Hydroxylgruppe des Zuckerteils Z mit einer Phosphorsäuregruppe esterartig verbunden ist, können die nichtphosphorylierten Verbindungen der Formel I nach jeder Methode phosphoryliert werden, die für die Herstellung einer P-O-C-Bindung geeignet ist.

In solchen Fällen, in denen eine Hydroxylgruppe des Zuckerteils reaktiver ist als die anderen Hydroxylgruppen im Zuckerring - dies ist z.B. die primäre Hydroxylgruppe am C-Atom 6 des Kohlenhydratteils -, kann die Phosphorylierung nach konventionellen Methoden, die für selektive Phosphorylierungen z.B. aus der Nucleotid- und der Kohlenhydratchemie bekannt sind, durchgeführt werden. Solch eine Methode beinhaltet allgemein die Umsetzung eines N-Alkyl-N-(aldohexopyranosyl)-carbonsäureamids, - harnstoffs, -carbamats oder -thiocarbamats mit einem Phosphorylierungsreagenz in einem organischen Lösungsmittel.

Als geeignetes Lösungsmittel kann eine Reihe von Solventien angesehen werden, z.B. Kohlenwasserstoffe wie Hexan, Cyclohexan oder Toluol, Halogenkohlenwasserstoffe wie Dichlormethan, Chlorhexan oder Chlorbenzol, Phenole, wie Phenol, Kresol, organische Säureester wie Essigester, Methylbenzoat, Nitroverbindungen wie Nitromethan, Nitroethan, Nitrobenzol, Nitrile wie Acetonitril, Malononitril, Ether wie Diethylether, Tetrahydrofuran, Dioxan, Ethylenglycoldimethylether, oder Trialkylphosphate wie Trimethylphosphat oder Triethylphosphat.

Diese Solventien können entweder allein oder als Mischung untereinander oder in Mischung mit einer organischen Base wie Pyridin, Triethylamin oder Picolin oder mit einem Salz aus einer organischen Base und einer anorganischen oder organischen Säure wie Pyridinhydrochlorid oder Pyridinium-para-Toluolsulfonat verwendet werden.

Als Phosphorylierungsreagenzen sind geeignet Phosphorhalogenide wie Phosphortrichlorid, Phosphoroxychlorid, Phenylphosphordichlorid, Diphenylphosphochloridat, Dibenzylphosphochloridat oder partiell hydrolysierte

Phosphoroxychloride. Als Beispiel für die Kombination von Reagenz und Solvens ist Phosphoroxydichlorid und Trialkylphosphat oder Dibenzylphosphochloridat und Dichlormethan-Toluol und Pyridin besonders geeignet.

Die Reaktionstemperatur liegt zwischen -20°C und +50°C, bevorzugt zwischen 0°C und Raumtemperatur, die Reaktionszeit liegt zwischen einigen Stunden und einigen Tagen.

In den Fällen, in denen eine selektive Phosphorylierung einer Hydroxylgruppe nicht durchgeführt werden kann, weil entweder eine weniger reaktive sekundäre Hydroxylgruppe des Zuckerrestes bei Vorhandensein einer reaktiven primären Hydroxylgruppe phosphoryliert werden soll, oder aber eine sekundäre selektiv bei Vorhandensein anderer sekundärer Hydroxylgruppen phosphoryliert werden soll, ist der eigentlichen Phosphorylierung eine Reihe von Schutzgruppenoperationen vorzuschalten, bei der selektiv die zu phosphorylierende Hydroxylgruppe am Ende der Blockierungsreaktionen frei, d.h. unsubstituiert, vorliegt. Die nicht zu phosphorylierenden Hydroxylgruppen müssen also vor der Phosphorylierung blockiert werden.

Geeignete Schutzgruppen für Zuckerderivate sind in der einschlägigen Literatur beschrieben (z.B. C.B. REESE, in: Protecting Groups in Org. Chem., 1973, p 95 - p 143; Plenum Press). Es können alle in der Zuckerchemie verwendeten Schutzgruppen und Kombinationen daraus benutzt werden.

Geeignete Schutzgruppen sind z.B. Ester wie Acetyl, Benzoyl, Pivaloyl, p-Methoxybenzoyl, Ether wie Benzyl, p-Methoxybenzyl, Allyl, 1-Propenyl, Alkylidenverbindungen wie Ethyliden, Isopropyliden, Benzyliden, Orthoester wie 1-Methoxy-ethyliden, 1-Ethoxyethyliden, Silylether wie Trimethylsilyl, t-Butyldimethylsilyl, Organometallverbindungen wie Borsäureester oder Zinnether oder Zinnketale wie Tributylstannyl oder Dibutylstannyliden.

Die durch diese Schutzgruppen blockierten Kohlenhydratderivate werden dann an der noch freien Hydroxylgruppe in einem geeigneten Lösungsmittel phosphoryliert. Geeignete Lösungsmittel und geeignete Verfahren zur Phosphorylierung sind oben genannt.

Zur Darstellung der freien phosphorylierten oder nicht phosphorylierten Verbindungen gemäß Formel I werden dann gegebenenfalls vorhandene Schutzgruppen nach den üblichen Methoden abgespalten.

Die Isolierung und Reinigung der gegebenenfalls mono- oder diphosphorylierten Glycosylamide, -carbamate, -thiocarbamate oder -harnstoffe gemäß Formel I erfolgt nach konventionellen Methoden.

Z.B. kann sie durchgeführt werden durch Auswahl oder Kombination der gängigen Reinigungsmethoden wie Adsorptionschromatographie an Kieselgel oder Ionenaustauscherharzen, Extraktion oder Kristallisation.

Die zuvor beschriebenen, glycolipidähnlichen Substanzen (nachfolgend GLA = Glycolipidanaloge genannt) sind pharmazeutisch wirksame Substanzen. So wird z.B. in der DE-OS 32 13 650 für solche Verbindungen eine Steigerung der körpereigenen Abwehr beschrieben.

Durch die Überführung der GLA in liposomale Form ist es möglich, feinteilige wäßrige Dispersionen der GLA zur Verfügung zu stellen, obwohl die GLA selbst praktisch unlöslich in Wasser sind.

Während bei konventionellen Liposomen-Konzepten die Phospholipide (als natürliche oder synthetisch hergestellte Membrankomponenten) zunächst die Liposomen-Matrix bilden, die dann mit Wirkstoffen gekoppelt wird (z.B. durch Einschluß wasserlöslicher Substanzen im wäßrigen Innenvolumen von Liposomen oder durch Einlagerung lipophiler Substanzen im lipophilen Teil der Liposomen-Membran oder Kopplung - kovalent oder adsorptiv - von Substanzen mit der Außen- oder Innenfläche von Liposomen) ist es ein besonderer Vorteil gemäß dieser Erfindung, daß die GLA selber die Wirkstoffe darstellen und gleichzeitig Bausteine der Liposomen sind, d.h. es besteht Identität von Wirkstoff und Liposomen-Matrix-Komponenten. Dadurch kann auf Formulierungshilfsstoffe völlig oder weitgehend verzichtet werden, was pharmakologisch von besonderem Interesse ist.

Zur Überführung der GLA in liposomale Form geht man im allgemeinen wie folgt vor.

Vorzugsweise wird zunächst der Feststoff (GLA, gegebenenfalls Mischungen von GLA und gegebenenfalls Hilfsstoffe) in einem geeigneten organischen Lösungsmittel gelöst. Das Lösungsmittel wird anschließend wieder abgedampft, so daß der Feststoff feinverteilt zurückbleibt. Hier haben sich Tetrahydrofuran, Chloroform und eine Mischung von Methanol und Methylenchlorid, vorzugsweise 1:9 (V/V) besonders bewährt. Auch Ethanol ist geeignet.

Danach kann die wäßrige Dispersion entweder durch Beschallung mit Ultraschall oder durch Dispergierung mit einem Schnellrührgerät erzeugt werden.

Bei der Ultrabeschallung geht man im allgemeinen so vor, daß die äußere wäßrige Phase vorgelegt wird. Der (wasserunlösliche) Feststoff bzw. das Feststoffgemisch wird dann zugegeben und die Mischung vorzugsweise mit einem Eintauch-Ultraschallgerät dispergiert bis eine milchig trübe bis bläulich opaleszierende Dispersion entsteht.

Besonders wichtig im Hinblick auf technische Verfahren ist die Dispergierung mit einem Schnellrührer. Diese Dispergierungsart ist von Interesse auch zur Erzeugung kleiner Liposomen-Teilchengrößen ( $\approx$ 100 nm).

Bei der Dispergierung nach beiden Methoden arbeitet man im allgemeinen bei Temperaturen von 10-90°C, vorzugsweise bei 40-90°C. Die optimale Dispergierungstemperatur für das einzelne GLA kann leicht durch entsprechende Reihenversuche ermittelt werden.

So haben sich beispielsweise folgende Dispergierungstemperaturen ergeben:

| Dispergierte Substanzen (GLA nach Beispiel) | optimale Dispergierungstemperatur °C |
|---|---|
| 2 | 50-70 |
| 1 | 50-70 |
| 8 | 50-70 |
| 5 | 80-90 |
| 6 | 80-90 |
| 4 | 40-70 |
| 7 | 80-90 |

Als wäßrige Phase bei der Dispergierung haben sich besonders die folgenden als zweckmäßig erwiesen:

ein Phosphatpuffer mit pH 5,0-8,5; oder eine Lösung von Mannit (1-10%) in Phosphatpuffer; oder eine 1-10%ige Mannit-Lösung; ebenso Phosphatpuffer, der etwa 1 % EDTA enthält; weiter isotone, phosphatgepufferte Kochsalzlösung, Tris-Puffer und Puffer + Lutrol (0-30 %).

Als weitere Zusatzstoffe kommen ferner in Betracht: Cholesterin, Phosphatidylcholin (Lecithin) und Phosphatidylethanolamin. Setzt man die eingesetzte GLA-Menge mit 10 Gewichtsteilen an, so können diese Zusatzstoffe im Bereich von 0-100 Gewichtsteilen eingesetzt werden.

Zur Präparation großer Volumina verwendet man zweckmäßig Durchlauf-Schnellrührer-Geräte, die eine hohe Pumpleistung entwickeln. Durch Anschluß beliebig großer, externer Wasser-Volumina kann damit im Durchfluß- und Rezirkulationsverfahren gearbeitet werden. Der Feststoff wird

im Schnellrührer-Einlauf zugegeben. Mit größerwerdenden Volumina verlängert sich die Dispergierzeit. Für ein 1 Liter-Volumen werden im allgemeinen ca. 30 min. benötigt, wobei diese Zeit geräteabhängig ist.

Will man höhere GLA-Konzentrationen in der Dispersion, etwa von mehr als etwa 1 mg/ml erzielen, so ist zu beachten, daß GLA-Liposomen dann häufig nicht mehr stabil sind. Deshalb ist es zweckmäßig, Hilfsstoffe zur Stabilisierung der GLA-Liposomen einzubauen. Vorzugsweise werden hierzu negativ geladene Phospholipide wie Phosphatidylserin, Phosphatidylglycerol, Dicetylphosphat, Phosphatidylinositol, Sphingomyelin sowie gesättigte und ungesättigte Fettsäuren (Palmitinsäure, Ölsäure, Linolsäure, Linolensäure) verwendet, aber auch neutrale und positiv geladene Lipide sind verwendbar. Diese Zusatzstoffe können einen relativen Gewichtsanteil von etwa 5-200 %, bezogen auf den Gewichtsteil der GLA, ausmachen; bevorzugt liegt dieser Gewichtsanteil im Bereich von 5-30 %. Wegen dieses bevorzugten, mengenmäßig geringen Anteils der Stabilisierungs-Additive besteht praktisch die GLA-Liposomenmatrix überwiegend aus Wirkstoff.

Wie bereits oben erwähnt, können auch Mischungen der GLA untereinander zur Herstellung von GLA-Liposomen verwendet werden.

Für pharmazeutische Anwendungen werden häufig hohe GLA-Konzentrationen benötigt. Hierfür ist es ein besonderer Vorteil, daß GLA-Liposomen unter Verwendung der oben erwähnten stabilisierenden Hilfsstoffe mit hoher GLA-Konzentration (z.B. 10% (w/v) und darüber) hergestellt werden können.

Ein anderes Verfahren zur Herstellung hoher Konzentrationen von GLA-Liposomen-Dispersionen besteht in einer Aufkonzentrierung durch Ultrafiltration. Auf diese Weise läßt sich die Konzentration auf das 10-fache der Ausgangskonzentration und darüber steigern, z.B. 100 mg/ml und darüber.

Die erfindungsgemäßen GLA-Liposomen haben sich in Tests als Teilchengrößen-stabil über einen Zeitraum von einem Jahr erwiesen. Die maximale Langzeitstabilität ist nicht bekannt. GLA-Liposomen sind aber generell wesentlich langzeitstabiler als viele Arten von Phospholipid-Liposomen.

Die GLA-Liposomen der Erfindung liegen im allgemeinen mit mittleren Teilchengrößen in der Dispersion von etwa 20 nm bis etwa 10.000 nm vor. Bevorzugte mittlere Teilchengrößen von 50-200 nm, lassen sich durch geeignete Wahl der Dispergierungsbedingungen gut erreichen.

Die Teilchengrößenbestimmung kann durch Elektronenmikroskopie oder dynamische Laser-Lichtstreuung erfolgen.

Es soll noch erwähnt werden, daß in einigen Fällen spontane Dispergierung auftreten kann.

Dies tritt insbesondere dann auf, wenn Ethanol/Wassermischungen als Dispergierungsmedium verwendet werden und wenn die GLA-Konzentration niedrig ist, z.B. ca. $\lesssim$ 10 mg/ml.

A) Herstellungsbeispiele für GLA

1. N-(D-Glucopyranosyl)-N-octadecyl-ölsäureamid

10 g N-Octadecyl-D-glucopyranosylamin werden in 80 ml Tetrahydrofuran aufgeschlämmt und nach Zusatz von 10 g Soda tropfenweise mit 7 g Ölsäurechlorid in 10 ml Tetrahydrofuran versetzt. Nach beendeter Umsetzung (dünnschichtchromatographische Kontrolle, Laufmittel $CH_2Cl_2$/$CH_3OH$ = 13:1) wird filtriert, das Filtrat im Vakuum eingedampft und das Rohprodukt säulenchromatographisch an Kieselgel (Laufmittel $CH_2Cl_2/CH_3OH$ = 15:1) gereinigt.

Rf-Wert: 0,53 in $CH_2Cl_2/CH_3OH$ = 13:2

2. N-(D-Glucopyranosyl)-N-octadecyl-laurinsäureamid

Herstellung analog Beispiel 1 aus 10 g N-Octadecyl-glucopyranosylamin und 5 g Dodecansäurechlorid

$\alpha_D$ = +8° (c = 1,0 in Dioxan)

3. N-(2-Desoxy-2-lauroylamido-D-glucopyranosyl)-N-octadecyl-laurinsäureamid

6,5 g D-Glucosamin-Hydrochlorid werden in 30 ml Isopropanol und 10 ml Wasser auf 60°C erwärmt

und mit 12,1 g Stearylamin versetzt. Nach 10 min. wird auf Raumtemperatur abgekühlt. Das auskristallisierte Produkt wird abfiltriert. Der Rückstand wird in 120 ml Tetrahydrofuran suspendiert und mit 20 g Natriumcarbonat versetzt. Zu dieser Mischung werden 12 g Dodecansäurechlorid in 20 ml Tetrahydrofuran zugetropft. Nach beendeter Reaktion wird wie in Beispiel 1 beschrieben aufgearbeitet und das entstandene Hauptprodukt säulenchromatographisch (Laufmittel $CH_2Cl_2/CH_3OH$ = 25:1) gereinigt.

Schmp. 78°C

4. N-(D-Galactopyranosyl)-N-tetradecyl-ölsäureamid

Herstellung analog Beispiel 1 aus 30 g D-Galactose, 53 g Tetradecylamin, 40 g Ölsäurechlorid

$\alpha_D$ = 11° (c = 1,0 in $CH_2Cl_2$)

5. N-(D-Mannopyranosyl)-N-octadecyl-laurinsäureamid

Herstellung analog Beispiel 2 aus 10 g N-Octadecyl-D-mannopyranosylamin und 5 g Dodecansäurechlorid

$\alpha_D$ = 11,3° (c = 1,1 in $CH_2Cl_2$)

6. <u>N-(D-Mannopyranosyl)-N-octadecyl-myristinsäureamid</u>

Herstellung analog Beispiel 2 aus 10 g N-Octadecyl-D-mannopyranosylamin und 5,5 g Tetradecansäurechlorid

$\alpha_D$ = 9,9° (c = 1,0 in Dichlormethan)

7. <u>N-(D-Mannopyranosyl)-N-tetradecyl-stearinsäureamid</u>

Herstellung analog Beispiel 2 aus 7,5 g N-Tetradecyl-D-mannopyranosylamin und 6 g Octadecansäurechlorid

$\alpha_D$ = 10,8° (c = 1,0 in Tetrahydrofuran)

8. <u>N-(2-Acetamido-2-desoxy-D-glucopyranosyl)-N-octadecyl-ölsäureamid</u>

15 g N-Acetylglucosamin und 18,8 g Dodecylamin werden in 50 ml Ethanol für 3 h unter Rühren auf 80°C erwärmt. Dann wird heiß von ungelösten Bestandteilen abfiltriert, das Filtrat abgekühlt, das ausgefallene Produkt abgesaugt und mit Ethanol und dann mit Ether nachgewaschen und im Vakuum getrocknet. 2,2 g des so erhaltenen N-(2-Acetamido-2-desoxy-D-glucopyranosyl)-do-

decylamins werden in 17 ml Tetrahydrofuran suspendiert, mit 2 g Soda versetzt und unter Rühren mit 1,45 g Ölsäurechlorid in 5 ml THF versetzt. Aufarbeitung wie in Beispiel 1 beschrieben.

$\alpha_D$ = 9,2° (c = 0,56 in Methanol)

9. N-(2-Acetamido-2-desoxy-D-glucopyranosyl)-N-dodecyl-laurinsäureamid

Herstellung analog Beispiel 8 aus N-Acetylglucosamin, Dodecylamin und Dodecansäurechlorid

Rf-Wert = 0,21 in $CH_2Cl_2/CH_3OH$ = 10:1

10. N-(2-Acetamido-2-desoxy-D-glucopyranosyl)-N-dodecyl-stearinsäureamid

Herstellung analog Beispiel 8 aus N-Acetylglucosamin, Dodecylamin und Octadecansäurechlorid

Rf-Wert = 0,23 in $CH_2Cl_2/CH_3OH$ = 10:1

11. N-(D-Glucopyranosyl)-N-dodecyl-heptadecansäureamid

Herstellung analog Beispiel 1 aus D-Glucose, Dodecylamin und Heptadecansäurechlorid

Rf-Wert = 0,26 in $CH_2Cl_2/CH_3OH$ = 12:1

12. N-(D-Galactopyranosyl-6-Phosphat)-N-dodecyl-octadecansäureamid

Gemäß Beispiel 1 wird aus D-Galactose, Dodecylamin und Octadecansäurechlorid die Verbindung N-(D-Galactopyranosyl)-N-dodecyl-octadecansäureamid hergestellt.

6,1 g dieser Verbindung werden in 60 ml Dichlormethan gelöst und mit 6 g Dibenzylphosphochloridat, gelöst in 10 ml Toluol, und 3,1 ml Pyridin versetzt. Nach Beendigung der Reaktion wird filtriert, das Filtrat im Vakuum eingedampft und säulenchromatographisch getrennt (Laufmittel Toluol/Ethanol = 10:1).

Das erhaltene Hauptprodukt (6 g) wird in 100 ml Tetrahydrofuran und 10 ml Eisessig gelöst und in Gegenwart von 2 g Palladium-Kohle (5 %ig) hydriert.

Nach beendeter Wasserstoffaufnahme wird vom Katalysator abfiltriert, das Filtrat im Vakuum zum Sirup eingedampft und mehrfach mit Toluol coevaporiert.

$\alpha_D$ = 1,2° (c = 1,0 in Dimethylformamid)

0155625

13. N-(2-Amino-2-desoxy-D-glucopyranosyl)-N-octadecyl-stearinsäureamid Hydroacetat

6,0 g 2-(Benzyloxycarbonylamino)-2-desoxy-D-glucopyranose wird in 25 ml Dimethylformamid gelöst und nach Zugabe von 6,4 g Octadecylamin für 3 h im Vakuum auf 80° erwärmt. Nach Abkühlen auf Raumtemperatur wird das erhaltene Glycosylamin in 50 ml Tetrahydrofuran und 5 ml Ethanol gelöst und nach Zugabe von 10 g Soda mit 7,3 g Stearinsäurechlorid, gelöst in 10 ml Tetrahydrofuran, tropfenweise versetzt. Nach beendeter Reaktion wird der Feststoff abfiltriert, das Filtrat im Vakuum eingedampft und der erhaltene Rückstand säulenchromatographisch gereinigt (Laufmittel $CH_2Cl_2/CH_3OH$ = 20:1). 9,5 g des erhaltenen Reinproduktes werden in 100 ml Dichlormethan, 100 ml Methanol und 50 ml Eisessig gelöst und in Gegenwart von 1 g Palladium-Kohle (5 %ig) hydriert. Nach beendeter Wasserstoffaufnahme wird vom Katalysator abfiltriert, im Vakuum eingeengt, mehrfach mit Toluol versetzt und eingedampft.

$\alpha_D$ = 7,0° (c = 0,90 in Tetrahydrofuran)

B) Herstellungsbeispiele für GLA in Liposomenform als feinteilige wäßrige Dispersion

14. 50 mg der Substanz nach Beispiel 2 und 5 mg Phosphatidylserin (PS) wurden in 2 ml eines

Methanol-Methylenchlorid-Gemisches (1:9) molekular gelöst. Das Lösungsmittelgemisch wurde im Rotationsverdampfer abgedampft, bis ein fester trockener Film an der Gefäßwand zurückblieb. Dem Gefäß mit dem trockenen Film wurden 10 ml einer wäßrigen Pufferlösung (0,01 mol/l Na-Phosphatpuffer, pH 7,3) zugegeben. Mit einem Eintauch-Ultraschallgerät (z.B. Branson B 12, Ultraschallfinger 1/2 Zoll, Leistungsstufe 4-5) wurde das wäßrige Medium im Gefäß mit dem festen Lipid-GLA-Film ca. 10 Min. lang beschallt, wobei die Temperatur der Flüssigkeit durch ein äußeres Wasserbad zwischen 50 und 60°C gehalten wurde. Durch die Beschallung löste sich der Lipid-GLA-Film von der Gefäßwand, und es bildete sich eine wäßrige Dispersion. Anschließend wurde die Dispersion ca. 20 Min. lang bei 1200 g zentrifugiert, wobei die vom Ultraschallfinger abgelösten Titan-Partikel sedimentieren. Der Überstand bildete die fertige wäßrige Dispersion. Die mittlere Teilchengröße betrug 110 nm.

15. Eine Lösung von 5 mg der Verbindung nach Beispiel 2 in 2 ml Methanol/Methylenchlorid (1:9) wurde hergestellt und nach Abdampfen des Lösungsmittels im Rotationsverdampfer wurde weiter verfahren wie Beispiel 14. Es wurde eine stabile wäßrige Dispersion erhalten. Mittlere Teilchengröße 95 nm.

16. Lösung von 50 mg der Verbindung nach Beispiel 2 und 5 mg Dicetylphosphat in 2 ml Methanol/ Methylenchlorid (1:9) und abdampfen im Rotationsverdampfer. Verfahren sonst wie Beispiel 14. Es wurde eine stabile wäßrige Dispersion erhalten. Mittlere Teilchengröße 126 nm.

17. Lösung von 50 mg der Verbindung nach Beispiel 1 und 5 mg PS in 2 ml Methanol/Methylenchlorid (1:9). Verfahren sonst wie Beispiel 14. Es wurde eine stabile wäßrige Dispersion erhalten. Mittlere Teilchengröße 93 nm.

18. Lösung von 50 mg der Verbindung nach Beispiel 8 und 5 mg PS in 2 ml Methanol/Methylenchlorid (1:9). Verfahren sonst wie Beispiel 14. Es wurde eine stabile wäßrige Dispersion erhalten. Mittlere Teilchengröße 87 nm.

19. Lösung von 50 mg der Verbindung nach Beispiel 5 und 5 mg PS in 2 ml Methanol/Methylenchlorid (1:9). Verfahren sonst wie Beispiel 14 mit der Ausnahme, daß die Temperatur des wäßrigen Mediums während der Beschallung zwischen 70 und 90°C gehalten wurde. Es wurde eine stabile wäßrige Dispersion erhalten. Mittlere Teilchengröße 88 nm.

20. Lösung von 50 mg der Verbindung nach Beispiel 6 und 5 mg PS in 2 ml Methanol/Methylenchlorid (1:9). Verfahren wie Beispiel 19 mit folgender Änderung, daß die Beschallung in einem handelsüblichen abgedichteten, druckfesten Gefäß unter ca. 2 Atm. Überdruck ($N_2$) ausgeführt wurde. Es wurde eine stabile wäßrige Dispersion erhalten. Mittlere Teilchengröße 135 nm.

21. Lösung von 50 mg der Verbindung nach Beispiel 4 und 5 mg PS in 2 ml Methanol/Methylenchlorid (1:9). Verfahren sonst wie Beispiel 14 mit der Ausnahme, daß die Beschallungstemperatur zwischen 40 und 60°C gehalten wurde. Es wurde eine stabile wäßrige Dispersion erhalten. Mittlere Teilchengröße 145 nm.

22. Lösung von 50 mg der Verbindung nach Beispiel 7 und 5 mg PS in 2 ml Methanol/Methylenchlorid (1:9). Verfahren sonst wie Beispiel 19. Es wurde eine stabile wäßrige Dispersion erhalten. Mittlere Teilchengröße 182 nm.

23. Lösung von 1 g der Verbindung nach Beispiel 2 und 100 mg PS in 40 ml Methanol/Methylenchlorid (1:9). Abdampfen des Lösungsmittels in einem Rotationsverdampfer oder durch Abblasen mit einem $N_2$-Strom. Zugabe von 200 ml eines wäßrigen

Mediums (0,01 mol/l Na-Phosphatpuffer, pH 7,3). Dispergierung mit einem Ultraschall-Eintauchgerät bis zur vollständigen Ablösung des GLA-PS-Films von der Gefäßwand. Anschließend Dispergierung mit einem Eintauch-Schnellrührer (Ultraturrax oder Polytron, ca. 20.000 Umdrehungen/Minute) bei Temperaturen im Bereich zwischen 50°C und 60°C für ca. 30 Minuten. Es wurde eine stabile wäßrige Dispersion erhalten. Mittlere Teilchengröße 130 nm.

24. Lösung von 1 g der Verbindung nach Beispiel 2 und 100 mg PS in 40 ml Methanol/Methylenchlorid (1:9). Abdampfen der Lösungsmittel bis zur Bildung eines trockenen, festen Films. Mechanisches Ablösen des festen GLA-PS-Films und grobe Zerkleinerung des Feststoffs. Anschließend Zugabe von 200 ml eines wäßrigen Puffermediums. Ca. 30 Min. lang Dispergierung mit einem Eintauch-Schnellrührer im Temperaturbereich zwischen 50 und 60°C. Es wurde eine stabile wäßrige Dispersion erhalten. Mittlere Teilchengröße 123 nm.

25. Lösung von 8 g der Verbindung nach Beispiel 2 und 1,6 g der Verbindung nach Beispiel 12 in 100 ml Methanol/Methylenchlorid (1:9). Sonst wie Beispiel 27 mit der Modifikation, daß die Dispergierungszeit mit dem Schnellrührer 1 h betrug. Es wurde eine stabile wäßrige Dispersion erhalten. Mittlere Teilchengröße 77 nm.

26. Lösung von 40 g der Verbindung nach Beispiel 2 und 8 g der Verbindung nach Beispiel 12 in 500 ml Methanol/Methylenchlorid (1:9). Abdampfen des Lösungsmittels bis zur Abscheidung eines trockenen Feststoffgemisches. Ablösen des Feststoffes und grobe mechanische Zerkleinerung bis zur Bildung eines schüttfähigen groben Pulvers. Benutzung eines gekapselten Durchlauf-Schnellrührers (Inliner, Baureihe Megatron 47/6, Fa. Kinematika) mit einem äußeren Vorratsgefäß, in dem sich 1 l einer wäßrigen Pufferlösung befanden. Das Vorratsgefäß hatte am Boden einen Auslauf, der sich über dem Einlauf des Schnellrührers befand und mit dem Schnellrührer-Einlauf verbunden war. Der Schnellrührer-Auslauf führte über eine Schlauchverbindung in den Einlauf des Vorratsgefäßes zurück. Das Vorratsgefäß wurde durch ein Temperierbad auf 40 bis 50°C eingestellt. Zugabe des Feststoff-Gemisches in das Vorratsgefäß bei schnellem Lauf des gekapselten Durchlauf-Rührers. Laufzeit des Rührers ca. 1 bis 2 h. Es wurde eine stabile wäßrige Dispersion erhalten. Mittlere Teilchengröße 90 nm.

27. Lösung von 18 g der Verbindung nach Beispiel 2 und 4,5 g Dicetylphosphat in 100 ml Ethanol. Abdampfen des Lösungsmittels bis zur Abscheidung eines trockenen Feststoffs bei 70°C. Ablösen des Feststoffs durch schnelle Abkühlung. Zugabe des Feststoffs zu 300 ml einer vorgelegten Lösung von 0,1 mol/l Tris-Puffer pH 8,0. Dispergierung mit einem Schnellrührer bei 70°C. Nach 30 Min. Dispergierzeit wird eine homogene, nichtsedimentierende Liposomendispersion erhalten. Mittlere Teilchengröße 110 nm.

28. Lösung von 0,6 g der Verbindung nach Beispiel 2 und 0,1 g Ölsäure in 5 ml Ethanol bei 60°C. Abdampfen des Lösungsmittels bei 60°C bis zur Abscheidung eines trockenen Feststoffs. Zugabe von 8,1 ml Wasser und 1,9 ml Natronlauge (0,1 mol/l). Ultraschall-Dispergierung und weitere Behandlung wie bei Beispiel 14. Die erhaltene Dispersion hat einen pH-Wert von 7,5; die mittlere Teilchengröße beträgt 170 nm.

Biologische Beispiele

Die Substanzen der vorliegenden Verbindungsklasse weisen eine ausgeprägte Abwehr-steigernde Wirkung auf. Es wurde gefunden, daß die Verbindungsklasse die Antikörpersynthese des Immunsystems Antigen-spezifisch steigert und darüber hinaus die unspezifische wirtseigene Abwehr verstärkt. Diese Ergebnisse wurden anhand der nachfolgenden Versuchsanordnungen erhalten. Hierbei wurden die in Tabelle 1 beschriebenen Liposomen-Formulierungen verwendet.

1. Steigerung der primären Immunität in vitro gegen Schafserythrozyten (SE).

Es ist experimentell möglich, die Entwicklung einer humoralen Immunantwort mit heterologen roten Blutzellen durch primäre Immunisation von Maus-Milzzellen in Suspensionskulturen in vitro einzuleiten (R.I. Mishell und R.W. Dutton, J. Exp. Med. 126, 423 (1967)). Hierzu wurden Balb/c Maus-Milzzellen für 5 Tage in Gegenwart von Antigen (SE) und der Testsubstanz kultiviert. Die Zellen werden geerntet, gewaschen und zusammen mit dem Antigen und Komplement in semisolidem Agar ausplattiert und für 2 Stunden bei 37°C inkubiert. (N.K. Jerne, A.A. Nordin und C. Henry, "Cell bound Antibodies", eds. Amos and Koprowski, Wistar Inst. Press, Philadelphia, USA, pp 109 (1963)). Die Antigen-Sensibilisierung von Maus-Lymphozyten in der Primärkultur resultiert in der Synthese und Freisetzung von Antikörper.

Die spezifischen, sezernierten Antikörper binden sich an das SE-Antigen und lysieren diese Zellen durch die Gegenwart des Komplements (Plaque-Bildung). Als Liposomen formulierte Substanzen der vorliegenden Verbindungsklasse sind in der Lage, dosisabhängig im Bereich 3-100 µg/ml die Zahl der Antikörper-bildenen Zellen zu steigern (Tabelle 2).

2. Steigerung der primären humoralen Immunität in vivo gegen das lösliche Antigen Ovalbumin

NMRI-Mäuse wurden mit einer suboptimalen Antigendosis (1 µg/Tier, Tag 0) subcutan (s.c.) immunisiert. Bei suboptimaler Antigenstimulation wurde nur eine geringe Zahl von Lymphozyten der Tiere zur Antikörpersynthese angeregt. Die zusätzliche Behandlung der Tiere mit Verbindungen der genannten Beispiele der vorliegenden Erfindung ist in der Lage, bei einer einmaligen Applikation von 10-30 mg/kg subcutan den Antikörpertiter im Serum der Tiere signifikant zu steigern. Die Antikörpertiterbestimmung erfolgte durch indirekte Hämagglutination am Tag 10. Der Effekt der Behandlung wird durch den geometrischen Mittelwert der $log_2$-Titer ausgedrückt (s. Tabelle 3).

Der immunstimulierende Effekt der genannten Verbindungen ist im Gegensatz zu anderen, z.B. bakteriellen Immunstimulantien wie LPS aus Gram-negativen Bakterien Antigen-abhängig, d.h. die Substanzen be-

0155625

wirken überraschenderweise nur in Verbindung mit einem antigenen Reiz (hier SE oder Ovalbumin) die Induktion der Antikörpersynthese. Sie haben im Gegensatz zu den erwähnten konventionellen Immunstimulantien keine mitogenen Eigenschaften.

3. Verträglichkeit

Obwohl Verbindungen der beschriebenen Art ihre potenzierende Wirkung an der Maus beispielsweise bereits nach einer Einzeldosis von 10 mg/kg i.p. oder s.c. entfalten, werden auch bei Applikation von 100 mg/kg keine toxischen Effekte beobachtet. Die genannten Stoffe verfügen deshalb über eine gute Verträglichkeit.

Die erfindungsgemäßen Verbindungen haben die Fähigkeit, einerseits bei Mischung mit einem Antigen dessen Immunogenität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphocyten zu aktivieren.

Die neuen Verbindungen können somit als Adjuvantien in Mischung mit Impfstoffen dazu benutzt werden, den Impferfolg zu verbessern und den durch Immunität vermittelten Infektionsschutz gegenüber bakteriellen, viralen oder parasitären Erregern zu steigern.

Weiterhin eignen sich die beschriebenen Verbindungen in Mischung mit verschiedensten Antigenen als Adjuvantien bei der experimentellen und industriellen Herstellung von Antiseren für Therapie und Diagnostik.

Darüber hinaus können die neuen Verbindungen auch ohne gleichzeitige Antigenzufuhr dazu benützt werden, bereits unterschwellig ablaufende Abwehrreaktionen bei Mensch und Tier zu fördern. Die Verbindungen eignen sich demnach besonders für die Stimulation der körpereigenen Abwehr, z.B. bei chronischen und akuten Infektionen oder bei selektiven (antigenspezifischen) immunologischen Defekten, sowie bei angeborenen, aber auch erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunsuppressiv wirkenden Stoffen auftreten. Die genannten Stoffe können somit vorzugsweise auch in Kombinationen mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Tabelle 1

Zusammensetzung der Liposomen-Formulierungen mit denen die in Tab. 2 und Tab. 3 zusammengefaßten Versuchsergebnisse erhalten wurden

| Liposomen-Formulierung | Substanz Beispiel 2 | Dicetyl-phosphat | Soja Lecithin | Cholesterin |
|---|---|---|---|---|
| | | mg/ml | | |
| L 1 | 5 | 0.5 | - | - |
| L 1 K | - | 0.5 | 5 | - |
| L 2 | 5 | 5 | - | 3 |
| L 2 K | - | 5 | 5 | 3 |

L 1, L 2 Wirkstoff-Liposomen
L 1 K, L 2 K Kontroll-Liposomen
L 1, L 1 K in 0,01 mol/l Na-Phosphatpuffer, pH = 7.5
L 2, L 2 K in 0.05 mol/l Na-Phosphatpuffer, pH = 7.5

Tabelle 2

In vitro Versuche zur Steigerung der Anzahl Plaque-bildender Zellen gegen Schafserythrozyten-Antigen durch Substanz Beispiel 2 in Liposomenformulierung; Kennzeichnung der verwendeten Liposomenformulierungen gemäß Tabelle 1.

| Liposomen Formulierung | Dosis Substanz Beispiel 2 (µg/ml) | | | | | |
|---|---|---|---|---|---|---|
| | 0 | 1 | 3 | 10 | 30 | 100 |
| | PFC/Kultur | | | | | |
| L 1 | 1760 | 2100 | 1420 | 5800 | 7840 | 9760 |
| L 1 K | 2220 (1) | | | | | |
| L 2 | | 2170 | 4560 | 2640 | 4280 | |
| L 2 K | 650 (1) | | | | | |

PFC: Plaque bildende Zellen

(1) Dosierung der Kontroll-Liposomen entsprechend der höchsten Dosierung von Wirkstoff-Liposomen

Tabelle 3

In vivo Versuche zur adjuvantiven Wirkung von Substanz Beispiel 2 in Liposomenformulierung (Applikationsweg subcutan) am Beispiel des Antigens Ovalbumin(1 µg/ Tier; subcutan); Kennzeichnung der verwendeten Liposomenformulierungen gemäß Tabelle 1

| Liposomen-Formulierung | Dosis Substanz Beispiel 2 (mg/kg) 0 | 3 | 10 | 30 |
|---|---|---|---|---|
| | $log_2$-Titer | | | |
| L 1 | 4.0 | 4.2 | 6.6 (2) | 6.6 (2) |
| L 1 K | 4.0 (1) | | | |
| L 2 | 4.8 | 6.4 (2) | 7.6 (2) | 8.4 (2) |
| L 2 K | 4.8 (1) | | | |

(1) Dosierung der Kontroll-Liposomen entsprechend der höchsten Dosierung von Wirkstoff-Liposomen

(2) $p < 0.008$

Patentansprüche

1. Verbindungen der allgemeinen Formel (I)

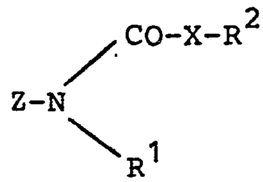

(I)

in der

$R^1$ einen gegebenenfalls substituierten geradkettigen oder verzweigten, gesättigten oder einfach oder mehrfach ungesättigten Alkyl- bzw. Alkenylrest mit bis zu 30 Kohlenstoffatomen,

$R^2$ Wasserstoff oder einen gegebenenfalls substituierten geradkettigen oder verzweigten, gesättigten oder ein- oder mehrfach ungesättigten Kohlenwasserstoffrest mit bis zu 30 Kohlenstoffatomen,

X eine Methylengruppe, ein Sauerstoffatom, ein Schwefelatom oder eine Gruppe NR, in der R für Wasserstoff oder eine Alkylgruppe mit bis zu 20 Kohlenstoffatomen steht und

Z einen gegebenenfalls phosphorylierten Glykosylrest, der über das anomere Kohlenstoffatom an den Amidstickstoff gebunden ist, bedeuten,

in Liposomenform in wäßriger Dispersion.

0155625

2. Verbindungen der Formel I gemäß Anspruch 1 in Liposomenform und einer mittleren Teilchengröße von 20 bis 10 000 nm.

3. Verfahren zur Herstellung von Liposomen aus Verbindungen der allgemeinen Formel I

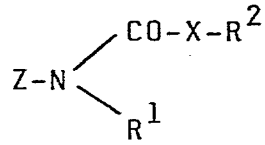

(I)

in der

$R^1$ einen gegebenenfalls substituierten geradkettigen oder verzweigten, gesättigten oder einfach oder mehrfach ungesättigten Alkyl- bzw. Alkenylrest mit bis zu 30 Kohlenstoffatomen,

$R^2$ Wasserstoff oder einen gegebenenfalls substuierten geradkettigen oder verzweigten, gesättigten oder ein- oder mehrfach ungesättigten Kohlenwasserstoffrest mit bis zu 30 Kohlenstoffatomen,

X eine Methylengruppe, ein Sauerstoffatom, ein Schwefelatom oder eine Gruppe NR, in der R für Wasserstoff oder eine Alkylgruppe mit bis zu 20 Kohlenstoffatomen steht und

Z einen gegebenenfalls phosphorylierten Glykosylrest, der über das anomere Kohlenstoffatom an den Amidstickstoff gebunden

ist, bedeuten

dadurch gekennzeichnet, daß man die Verbindungen der Formel I gegebenenfalls mit weiteren Hilfs- und Zusatzstoffen, in ein geeignetes wäßriges Medium gibt und durch Behandlung mit Ultraschall oder durch schnelles Rühren in eine wäßrige Dispersion, in der die Verbindungen in Liposomenform vorliegen, überführt.

4. Arzneimittel, enthaltend mindestens eine der Verbindungen der allgemeinen Formel I

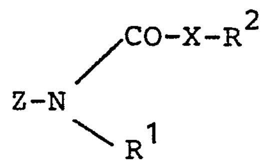

(I)

in der

$R^1$ einen gegebenenfalls substituierten geradkettigen oder verzweigten, gesättigten oder einfach oder mehrfach ungesättigten Alkyl- bzw. Alkenylrest mit bis zu 30 Kohlenstoffatomen,

$R^2$ Wasserstoff oder ein gegebenenfalls substituierten geradkettigen oder verzweigten, gesättigten oder ein- oder mehrfach ungesättigten Kohlenwasserstoffrest mit bis zu 30 Kohlenstoffatomen,

X eine Methylengruppe, ein Sauerstoffatom, ein Schwefelatom oder eine Gruppe NR, in der R für Wasserstoff oder eine Alkylgruppe mit bis zu 20 Kohlenstoffatomen steht, und

Z einen gegebenenfalls phosphorylierten Glykosylrest, der über das anomere Kohlenstoffatom an den Amidstickstoff gebunden ist, bedeuten

in Liposomenform in wäßriger Dispersion.

5. Parenteral applizierbare Arzneizubereitung nach Anspruch 4, welche Liposomen mit einem mittleren Durchmesser von 20 -10000 nm enthält, wobei die Liposomenmembran folgende Bestandteile aufweist:

a) 10 Gewichtsteile einer glycolipidähnlichen Substanz der Formel I

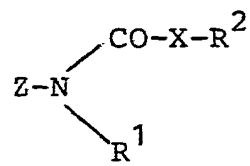

(I)

in der

$R^1$ einen gegebenenfalls substituierten geradkettigen oder verzweigten, gesättigten oder einfach oder mehrfach ungesättigten Alkyl- bzw. Alkenylrest mit bis zu 30 Kohlenstoffatomen,

$R^2$ Wasserstoff oder einen gegebenenfalls substituierten geradkettigen oder verzweigten, gesättigten oder ein- oder mehrfach ungesättigten Kohlenwasserstoffrest mit bis zu 30 Kohlenstoffatomen,

X eine Methylengruppe, ein Sauerstoffatom, ein Schwefelatom oder eine Gruppe NR, in der R für Wasserstoff oder eine Alkylgruppe mit bis zu 20 Kohlenstoffatomen steht, und

Z einen gegebenenfalls phosphorylierten Glykosylrest, der über das anomere Kohlenstoffatom an den Amidstickstoff gebunden ist, bedeuten,

b) 0 - 100 Gewichtsteile Lipide,

und wobei die Liposomen in einem wäßrigen Medium verteilt sind, welches einen pH-Wert von 5,0-8,5 aufweist.

6. Arzneimittelzubereitung gemäß Anspruch 5 enthaltend Liposomen mit einem mittleren Durchmesser von 50-200 nm.

7. Verfahren zur Herstellung von Arzneizubereitungen gemäß den Ansprüchen 4 und 5, dadurch gekennzeichnet, daß man 10 Gewichtsteile der glycolipidähnlichen Substanz und 1 - 100 Gewichtsteile Lipin in organischen Lösungsmitteln löst, anschließend das Lösungsmittel entfernt und den Rückstand nach Zugabe eines wäßrigen Mediums mit einem pH-Wert von 5,0 bis 85 bei Temperaturen zwischen 10 und 90°C dispergiert.

8. Verfahren gemäß Anspruch 7, dadurch gekennzeichnet, daß man als organische Lösungsmittel Methanol, Ethanol, Aceton oder leicht flüchtige Halogen-Kohlenwasserstoffe verwendet.

9. Verfahren gemäß Anspruch 7, dadurch gekennzeichnet, daß der Rückstand nach Entfernung des organischen Lösungsmittels durch Ultraschall dispergiert wird.

10. Verwendung der Verbindungen der allgemeinen Formel

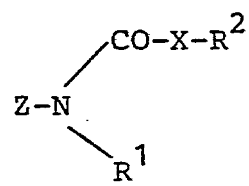

(I)

in der

$R^1$ einen gegebenenfalls substituierten geradkettigen oder verzweigten, gesättigten oder einfach oder mehrfach ungesättigten Alkyl- bzw. Alkenylrest mit bis zu 30 Kohlenstoffatomen,

$R^2$ Wasserstoff oder einen gegebenenfalls substituierten geradkettigen oder verzweigten, gesättigten oder ein- oder mehrfach ungesättigten Kohlenwasserstoffrest mit bis zu 30 Kohlenstoffatomen,

X eine Methylengruppe, ein Sauerstoffatom, ein Schwefelatom oder eine Gruppe NR, in der R für Wasserstoff oder eine Alkylgruppe mit bis zu 20 Kohlenstoffatomen steht, und

Z einen gegebenenfalls phosphorylierten Glykosylrest, der über das anomere Kohlenstoffatom an den Amidstickstoff gebunden ist, bedeuten, bei der Bekämpfung von Krankheiten.